# EUROPEAN PATENT APPLICATION

(11) **EP 4 616 851 A1**
(43) Date of publication of application: **17.09.2025**
(21) Application number: 24382278.0
(22) Date of filing: 14.03.2024
(51) Int. Cl.: A61K 9/20, A61K 31/4184, A61K 31/7048

(54) **PHARMACEUTICAL COMPOSITION COMPRISING A FIXED DOSE COMBINATION OF IVERMECTIN AND ALBENDAZOLE**

(71) Applicant: Laboratorios Liconsa, S.A., 28805 Alcalá de Henares (ES)
(72) Inventor: GERGIC HERMIDA, Esteban Pablo, Azuqueca de Henares (ES); SGARELLA CAROL, Nicolas, Azuqueca de Henares (ES); DE ALGORTA DE PINEDA, Jaime, Madrid (ES); KROLEWIECKI, Alejandro, Caba (AR)
(74) Representative: Schön, Christoph

(57) **Abstract**

The present invention relates to the field of treatment of parasites, in particular to a method of treating subjects suffering from soil transmitted helminths by administering a pharmaceutical composition comprising ivermectin and a benzimidazole carbamate formulated as a fixed-dose combination, a pharmaceutical composition in the form of a fixed-dose combination comprising ivermectin and benzimidazole carbamate, said composition for use in the treatment of subjects suffering from parasites and a method for producing a fixed-dose combination comprising ivermectin and benzimidazole carbamate.

## Description

### Field of the invention

The present invention relates to the field of treatment of parasites, in particular to a method of treating subjects suffering from infections with soil transmitted helminths (SHTs) by administering a pharmaceutical composition comprising ivermectin and a benzimidazole carbamate, a pharmaceutical composition in the form of a fixed-dose combination comprising ivermectin and a benzimidazole carbamate, said pharmaceutical composition for use in the treatment of subjects suffering from parasites infections and a method for producing a pharmaceutical composition comprising ivermectin and a benzimidazole carbamate.

### Background of the invention

Ivermectin is a member of the avermectin class, which has been shown in immunopharmacological studies to exert anti-inflammatory effects by inhibiting lipopolysaccharide-induced production of inflammatory cytokines, such as tumor necrosis factor alpha and interleukin(IL)-1ß, while upregulating the anti-inflammatory cytokine IL-10. It is a semi-synthetic derivative isolated from the fermentation of *Streptomyces avermitilis*, that belongs to the avermectin family of macrocyclic lactones. Ivermectin is a mixture containing 5-O-demethyl-22,23-dihydroavermectin A1a plus 5-O-demethyl-25-de(1-methylpropyl)-25-(1-methylethyl)-22,23-dihydroavermectin A1a, generally referred to as 22,23-dihydroavermectin B1a and B1b or H2B1a and H2B1b, respectively. The respective empirical formulas of H2B1a and H2B1b are C₄₈H₇₄O₁₄ and C₄₇H₇₂O₁₄ with molecular weights of 875.10 and 861.07, respectively.

Ivermectin is a macrocyclic lactone derivative, its therapeutic effect is thought to be prominently due to its anti-inflammatory properties, similar to that of other macrolides. Ivermectin has been reported to exert anti-inflammatory effects by inhibiting lipopolysaccharide-induced production of inflammatory cytokines. In addition to its anti-inflammatory mode of action, ivermectin possesses antiparasitic properties. Its predecessor, avermectin, is an antiparasitic agent of agricultural importance first isolated in 1974. Several studies support the role of ivermectin in the effective oral treatment of cutaneous demodicidosis (in combination with topical permethrin cream) and scabies, as well as topical treatment of head lice. Ivermectin causes death of parasites, primarily through binding selectively and with high affinity to glutamate-gated chloride channels, which occur in invertebrate nerve and muscle cells. This leads to the interruption of nerve impulses, causing paralysis and death of parasitic organisms. Ivermectin is known to act on Demodex mites in localized and generalized demodicidosis in animals and in humans.

At present in the US, Brazil and the EU oral tablets comprising 3 mg of ivermectin (see for example Stromectol^{®}, NDA 050742, Merck Sharpe and Dohme) are approved inter alia for the treatment of Onchoceriasis and Strongyloidiasis of the intestinal tract.

Furthermore, ivermectin has previously been studied as a therapeutic option for viral infections with in vitro data showing some activity against a broad range of viruses, including HIV, Dengue, Influenza and Zika virus.

Benzimidazole carbamates describe a novel class of anthelmintic and antiprotozoal agents which have been used in the treatment of a variety of intestinal parasite infections. Typically, benzimidazole carbamates such as mebendazole and albendazole show a good safety and efficacy profile and have been successfully used to treat helminth infections caused by A. lumbricoides with a cure rate of over 90 %. This treatment effect is taught to be achieved by larvicidal, ovicidal and vertical activities mainly exerted in the intra-intestinal region inter alia due to the poor intestinal absorption rate of <5% and the extensive first pass metabolism.

More than a quarter of the world population is at risk of infection with soil-transmitted helminths (STH), having the highest prevalence in regions with a warm and moist climate and in areas of the lowest socioeconomic status with poor sanitation and hygiene, which facilitates a transmission of STH. These diseases are the most prevalent of all neglected tropical diseases worldwide and disproportionately affect impoverished populations, causing significant morbidity in pre-school and school-age children (Jourdan et al., "Soil transmitted Helminth Infections", Lancet, vol. 6736 (17), 2017, pages 1-14). The 2017 Global Burden of Disease report ranks STH as the disease that poses the greatest burden of years lived with disability, with an estimation of 1.66 million years ("Global, Regional, and National Incidence, Prevalence, and Years Lived with Disability for 354 Diseases and Injuries for 195 Countries and Territories, 1990-2017: a Systematic Analysis for the Global Burden of Disease Study 2017", Lancet, vol. 392 (10159), 2018, pages 1789-185).

Four species of nematodes are collectively referred to as soil-transmitted helminths: the roundworm, *Ascaris lumbricoides*; the whipworm, *Trichuris trichiura*; and the hookworms, *Necator americanus* and *Ancylostoma duodenale.* These four species are frequently considered together because infection is diagnosed by the same laboratory method and treated with the same drugs. The nematode *Strongyloides stercoralis* can also be considered a soil-transmitted helminth, but, because special laboratory methods are used for proper diagnosis and a different class of medicine is used in its treatment, it is not included within the STH group.

In addition to interventions to improve sanitation and hygiene measures, the core intervention for reducing morbidity and transmission of STH is the preventive chemotherapy as periodic mass drug administration campaigns (Gabrielli et al., "Preventive Chemotherapy in Human Helminthiasis: Theoretical and Operational Aspects", Trans. R. Soc. Trop. Med. Hyg., vol. 105, 2011, pages 683-693). This implies a large-scale distribution of anti-helminthic drugs, typically with a single dose, to populations at risk without a previous diagnosis.

### Brief description of the invention

In view of said situation, it is an object of the present invention to provide a pharmaceutical composition in the /form of a fixed-dose combination comprising ivermectin and a benzimidazole carbamate which can be used in treating subjects suffering from infections with parasites, preferably soil-transmitted helminths (STHs).

It is a further object of the present invention to provide a pharmaceutical fixed-dose composition which can effectively and safely be administered to adults and children to treat infections with parasites such as soil-transmitted helminths (STHs) including *Ascaris lumbricoides*; *Trichuris trichiura*; *Necator americanus* and *Ancylostoma duodenale* and S. *stercoralis,* without creating any severe adverse side effects.

Moreover, to simplify an administration especially in countries in which access to clean water is restricted, it is advantageous that pharmaceutical compositions can be administered in a safe and simple manner to both adults and children. Therefore, it is a further object of the present invention to provide a pharmaceutical composition which allows a simple administration including an easy treatment regimen.

Furthermore, the formulation of a pharmaceutical fixed-dose combination which can be orally administered, can be challenging as such formulations often tend to show a reduced stability under warm and moist conditions in countries in which parasitic infections e.g. by soil-transmitted helminths have a high prevalence.

Thus, a further object of the present invention is to provide a pharmaceutical fixed-dose composition comprising ivermectin and a benzimidazole carbamate which can be administered to adults and children without water or other liquids and which pharmaceutical composition shows a high stability over a long period of time such as a period of at least 6 months.

Furthermore, another object of the present invention is to provide a method of treating subjects suffering from parasites, in particular a method of treating subjects suffering from infections with soil-transmitted helminths (STHs) in a safe (absence of adverse events) and efficacious manner which can avoid a risk of drug resistance by administering a pharmaceutical composition comprising ivermectin and a benzimidazole carbamate formulated as a fixed-dose combination.

### Detailed description of the invention

As used herein, the term "subject" means any animal, preferably a mammal, most preferably a human, to whom will be or has been administered compounds or unit dosage forms according to embodiments of the invention. Preferably, a subject is in need of, or is an object of observation or treatment or prevention of a parasitic infection, preferably a soil-transmitted helminthic infection e.g. caused by one or more of *Ascaris lumbricoides*; *Trichuris trichiura*; *Necator americanus* and *Ancylostoma duodenale* and *S*. *stercoralis.*

In one embodiment, "treatment" or "treating" refers to an amelioration, prophylaxis, or reversal of a disease or disorder, or of at least one discernible symptom thereof. In another embodiment, "treatment" or "treating" refers to an amelioration, prophylaxis, or reversal of at least one measurable physical parameter related to the disease or disorder being treated, not necessarily discernible in or by the mammal. In yet another embodiment, "treatment" or "treating" refers to inhibiting or slowing the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

The severity of an active parasitic infection can easily be determined for example by measuring the number of parasitic eggs per gram in a stool sample of a subject suffering from said parasitic infection. Hereby, the term "egg reduction rate" is often used in the respective field of the art in order to categorize the effectiveness of a medical treatment. Said "egg reduction rate" can be determined by calculating the number of eggs per gram in a stool sample obtained from the subject before medical treatment and the number of eggs per gram of a stool sample of said subject after medical treatment for e.g. 14 to 28 days, preferably 21 days, by microscopy. For an analysis of a stool sample by microscopy according to the present invention usually stool samples of 3 g or more are used. The stool samples may be subjected to a concentration procedure, if required, and subsequently investigated under the microscope in order to count the number of eggs in a stool sample.

In a first aspect the present invention provides an oral pharmaceutical composition in the form of a fixed-dose combination comprising ivermectin or a pharmaceutically acceptable salt or solvate thereof, a benzimidazole carbamate or a pharmaceutically acceptable salt or solvate thereof and at least one pharmaceutically acceptable excipient, wherein said oral pharmaceutical composition comprises ivermectin or a pharmaceutically acceptable salt or solvate thereof in an amount of 5 to 25 mg.

The oral pharmaceutical compositions according to the present invention show a very good activity in treating subjects suffering from infections with soil-transmitted helminths, an excellent stability over a long period of time, a good content uniformity and good dissolution and disintegrating properties.

In the following, when referring to a benzimidazole carbamate, the term a benzimidazole carbamate should mean - unless stated otherwise - that a benzimidazole carbamate or a pharmaceutically acceptable salt or a solvate thereof may be used.

Furthermore, when referring to ivermectin, the term ivermectin should mean - unless stated otherwise - that ivermectin or a pharmaceutically acceptable salt or a solvate thereof may be used.

With respect to the active ingredient ivermectin, present in said oral pharmaceutical composition, it is further well known in the respective field of the art that ivermectin may be used as amorphous ivermectin or crystalline ivermectin.

According to the present invention it was found that in the oral pharmaceutical composition of the present invention, especially in an immediate-release oral pharmaceutical composition preferably crystalline ivermectin particles should be present.

Said crystalline ivermectin particles for example may be produced by dissolving crude ivermectin in ethanol and adding formamide to the ethanol solution. Subsequently water should be added to said solution and the resulting solution should be cooled for precipitation.

After collecting the crystalline ivermectin particles, the wet crystalline ivermectin particles may be further slurred in a mixture of ethanol and water, isolated by filtration or centrifugation, dried and sieved so as to produce crystalline ivermectin particles.

According to the present invention it is preferred to use crystalline ivermectin particles having a particle size distribution in a range of X10 ≤ 40 µm, X50 ≤ 55 µm and/or X90 ≤ 150 µm, preferably of X10 ≤ 20 µm, X50 ≤ 35 µm and/or X90 ≤ 90 µm.

According to the present invention it was found that in the oral pharmaceutical composition of the present invention, especially in an immediate-release oral pharmaceutical composition preferably crystalline particles of a benzimidazole carbamate or a pharmaceutically acceptable salt or a solvate thereof should be present.

According to the present invention it is preferred to use crystalline benzimidazole carbamate particles having a particle size distribution in a range of X90 ≤ 30 µm, preferably of X90 ≤ 10 µm.

According to the present invention the oral pharmaceutical composition in the form of a fixed-dose combination comprises ivermectin or a pharmaceutically acceptable salt or solvate thereof in an amount of 5 to 25 mg, preferably in an amount of 5 to 21 mg, more preferably 7 to 20 mg, more preferably 8 to 19 mg, even more preferably 9 to 18 mg, most preferably 9 or 18 mg.

Usually, the ivermectin or a pharmaceutically acceptable salt or solvate thereof can be present in the oral pharmaceutical composition in the form of a fixed-dose combination according to the present invention in an amount of about 0.5% by weight to about 3% by weight, for example about 0.5% to about 2%, or about 0.6% to about 1.8%, or about 0.75% by weight to 1.5% by weight based on the total weight of the oral pharmaceutical composition.

In a preferred embodiment the oral pharmaceutical composition in the form of a fixed-dose combination comprises a benzimidazole carbamate or a pharmaceutically acceptable salt or solvate thereof in an amount of an amount of 200 to 800 mg, more preferably 250 to 600 mg, even more preferably 350 to 450 mg, most preferably 400 mg.

Usually, the benzimidazole carbamate or a pharmaceutically acceptable salt or solvate thereof can be present in the pharmaceutical composition of the oral solid unit dosage form according to the present invention in an amount of about 0.5% to about 70%, for example about 10% to about 60%, or about 15% to about 45%, or about 25% to 35% by weight based on the total weight of the oral solid unit dosage form.

In a preferred embodiment of the present invention the oral pharmaceutical composition in the form of a fixed-dose combination comprises as the two active ingredients (a) ivermectin and (b) albendazole as the benzimidazole carbamate.

In a preferred embodiment of the first aspect, the oral pharmaceutical composition in the form of a fixed-dose combination comprising ivermectin and a benzimidazole carbamate, preferably albendazole, is an immediate-release pharmaceutical composition in a unit dosage form. The immediate-release pharmaceutical composition in the form of a fixed-dose combination can be a pharmaceutical composition in the form of tablets, granules, capsules, powder, suspension, or liquid.

In a further preferred embodiment of the first aspect, the oral pharmaceutical composition in the form of an immediate-release fixed-dose combination comprising ivermectin and a benzimidazole carbamate, preferably albendazole, in a unit dosage form can be in the form of immediate-release tablets, such as oro-dispersible immediate-release tablets etc., wherein the immediate-release tablets do not comprise any release retarding agent.

The solubility characteristics of the active ingredients, i.e. (a) ivermectin or a pharmaceutically acceptable salt or solvate thereof and (b) the benzimidazole carbamate or a pharmaceutically acceptable salt or solvate thereof, preferably albendazole or a pharmaceutically acceptable salt thereof, in the immediate-release unit dosage forms according to the present invention are in accordance with the USP specification for immediate-release unit dosage forms containing ivermectin or a pharmaceutically acceptable salt or solvate thereof as the active ingredient and for immediate-release unit dosage forms containing a benzimidazole carbamate, preferably albendazole or a pharmaceutically acceptable salt or solvate thereof. For example, according to the present invention in the case of (a) ivermectin and (b) a benzimidazole carbamate (preferably albendazole) containing immediate-release unit dosage forms, usually more than 75%, preferably more than 80%, more preferably more than 90% of the two active ingredients ivermectin and benzimidazole carbamate, respectively, contained in the unit dosage forms can be dissolved in the dissolution medium within 45 minutes during agitation under appropriate conditions when using an USP device 2 (paddle) at 50 rpm in 900 ml of 0.01 M phosphate buffer, pH 7 with 0.5% of sodium lauryl sulfate for ivermectin and when using an USP device 2 (paddle) at 50 rpm in 900 ml of 0.1 N HCl, pH 1.2 with 1% of sodium lauryl sulfate for albendazole, respectively (see also USP43-NF38).

It is particularly preferred that the immediate-release pharmaceutical composition in the form of a fixed-dose combination comprising ivermectin and albendazole is an immediate-release tablet, such as an oro-dispersible immediate-release tablet.

According to the present invention immediate-release tablets according to the present invention are characterized in that the immediate-release tablets usually are disintegrated or dissolved in the mouth in contact with saliva without chewing, in less than 480 seconds, preferably less than 300 seconds, more preferably less than 180 seconds, more preferably less than 120 seconds, more preferably less than 60 seconds and most preferably less than 40 seconds, to form a particle suspension that can be swallowed.

In a further preferred embodiment of the first aspect, the oral pharmaceutical composition in the form of a fixed-dose combination comprising ivermectin and a benzimidazole carbamate, preferably albendazole, is an immediate-release unit dosage form for use in a treatment of parasitic infections, preferably soil-transmitted helminths. The immediate-release pharmaceutical composition in the form of a fixed-dose combination can be in the form of tablets, granules, capsules, powder, suspension, or liquid, more preferably in the form of immediate-release tablets such as oro-dispersible immediate-release tablets.

Examples of the at least one pharmaceutical excipient which can be used in preparing an immediate-release unit dosage pharmaceutical composition in the form of a fixed-dose combination comprising ivermectin and a benzimidazole carbamate include, but are not limited to:
(i) binders/fillers such as mannitol, crospovidone, croscarmellose sodium, polyvinyl acetate, povidone, such as povidone-30K or povidone-90K, starch, microcrystalline cellulose, lactose, dicalcium phosphate, and or combinations thereof;
(ii) disintegrants such as starch, croscarmellose sodium, sodium starch glycolate, and cross-linked polyvinyl pyrrolidone;
(iii) glidants such as silica and talc;
(iv) lubricants such as stearic acid, magnesium stearate, and sodium stearyl fumarate;
(v) sequestering agents and/or chelating agents such as ethylene diamine tetraacetic acid (EDTA), acetate or anhydrous citric acid;
(vi) colorants such as FD&C lake pigments;
(vii) flavoring agents such as natural and artificial flavors;
(viii) preservatives such as benzoic acid;
(ix) antioxidants such as butylated hydroxyanisole, butylated hydroxytolulene and citric acid;
(x) pH modifiers such as citric acid and fumaric acid.

Suitable further pharmaceutically acceptable excipients include other fillers, binders, disintegrants, lubricants, and the like, as is well known to a person skilled in the art. In preferred embodiments the immediate-release unit dosage forms are produced by compression.

Fillers suitable for use in manufacturing oral pharmaceutical compositions in the form of fixed-dose combinations include sugars including dextrose, sucrose, maltose and lactose; water-soluble compressible carbohydrates such as starch hydrolysates including sugaralcohols including mannitol, sorbitol, maltitol, xylitol, and dextrin and maltodextrin (e.g., microcrystalline cellulose or other cellulose derivatives), water insoluble, brittle materials (e.g., dicalcium phosphate, tricalcium phosphate, etc.), and mixtures thereof, preferably fillers selected from the group consisting of mannitol, croscarmellose sodium, carboxymethyl cellulose, microcrystalline cellulose and povidone, more preferably fillers selected from the group consisting of mannitol and croscarmellose sodium and mixtures thereof.

For example, commercially available mixtures of fillers which may be used according to the present invention include for example Ludiflash or Parteck ODT. Parteck ODT according to the present invention defines a composition comprising 95 wt% of mannitol and 5 wt% of croscarmellose sodium. Commercially available Ludiflash according to the present invention defines a composition comprising 84.0-92.0% D-mannitol, 4.0-6.0% Kollidon^{®} CL-SF, 3.5-6.0% polyvinyl acetate, 0.5-2.0% water and 0.25-0.60% povidone.

Usually, fillers may be present in an amount from about 5% to about 98% by weight, preferably from 10% to 95% by weight, and more preferably from 15% to 90% by weight based on the total weight of the oral pharmaceutical composition as the solid unit dosage form.

Suitable binders for preparing unit dosage forms by compression methods include anhydrous binders (e.g., polyvinylpyrrolidone, hydroxypropylmethylcellulose, and the like); wetting agents (such as acacia, alginate, agar, guar gum, low-cost soybeans, carrageenan, carboxymethyl cellulose, tara, gum arabic, tragacanth, pectin, xanthan gum, gellan gum, gelatin, maltodextrin, galactomannan, water-soluble compounds including fumaric acid, derivatives and mixtures thereof.

Usually, binders may be present in an amount from about 0.5% to about 30% by weight, preferably from 1% to 25% by weight, and more preferably from 3% to 20% by weight based on the total weight of the oral pharmaceutical composition as the solid unit dosage form.

Suitable disintegrants for preparing unit dosage forms by compression methods include sodium starch glycolate, crosslinked polyvinylpyrrolidone, crosslinked carboxymethylcellulose, crosscarmellose sodium, starch and microcrystalline cellulose and the like, preferably crosscarmellose sodium.

Usually, disintegrating agents may be present in an amount from about 2% to about 50% by weight, preferably from about 3% to about 45% by weight, and more preferably from 4% to 30% by weight based on the total weight of the oral pharmaceutical composition as the solid unit dosage form.

Suitable lubricants for preparing unit dosage forms by compression methods include long chain fatty acids and salts thereof such as magnesium stearate and stearic acid, talc, glycerides and waxes.

The lubricant may be present in an amount from about 0% to 5% by weight, preferably from about 0% to about 3% by weight based on the total weight of the oral pharmaceutical composition as the solid unit dosage form.

Suitable glidants for producing unit dosage forms by compression methods include colloidal silicon dioxide and the like.

Pharmaceutically acceptable adjuvants suitable for preparing unit dosage forms by compression methods usually include preservatives, high-concentration sweeteners (such as aspartame, acesulfame potassium, scallalose and saccharin), flavoring agents, coloring agents, antioxidants, surfactants and wetting agents, and the like, and mixtures thereof.

Although any pharmaceutically acceptable excipient as defined above or a combination thereof can be used as an excipient in producing an oral pharmaceutical composition as the solid unit dosage form according to the present invention, a preferred immediate-release pharmaceutical unit dosage form of the present invention contains in addition to the two active ingredients, at least one of the above-mentioned pharmaceutically acceptable filler(s) and/or disintegrant(s).

According to the present invention it was surprisingly found that a preferred immediate-release pharmaceutical unit dosage form of the present invention shows an improved stability, if said immediate-release pharmaceutical unit dosage form contains a total amount of the filler and disintegrant in a range of 35 to 90 wt% based on the total weight of the oral pharmaceutical composition, preferably in a range of 45 to 80 wt% based on the total weight of the oral pharmaceutical composition, more preferably in a range of 50 to 77 wt% based on the total weight of the oral pharmaceutical composition and most preferably in a range of 55 to 75 wt% based on the total weight of the oral pharmaceutical composition.

As degradation products of the active ingredients can impact the safety of the pharmaceutical composition, it is of specific interest to keep the amounts of impurities caused by degradation at a minimum level. Hence, it is desirable that the pharmaceutical composition comprising ivermectin and a benzimidazole carbamate meets the following criteria:
Ivermectin:
   Hydroxyoxy impurity: NMT (= Not More Than) 0.5%
   Any unknown impurity: NMT 0.2%
   Total impurities: NMT 5%
Benzimidazole carbamate:
   Unknown maximum individual impurity: NMT 0.2%
   Total degradation products: NMT 1.3%

It is preferred that the pharmaceutical composition comprising ivermectin and a benzimidazole carbamate comprises low amounts of impurities after storage for 12 months at 25°C/60% RH or 6 months at 40°C/75% RH, which is an amount of total degradation products of not more than (NMT) 1.0% by weight, e.g. NMT 0.5% by weight, preferably NMT 0.2% by weight based on the weight of the total pharmaceutical composition.

In view of an improved stability, dissolution and rapid disintegration as well as good content uniformity, in a preferred embodiment of the present invention the immediate-release oral pharmaceutical composition is an immediate-release tablet comprising a fixed-dose combination of ivermectin and albendazole as a benzimidazole carbamate, wherein said immediate-release tablet comprises at least one excipient selected from the group of mannitol and/or croscarmellose sodium, as filler and/or disintegrant, wherein the total amount of mannitol and/or croscarmellose sodium is in a range of 37 to 88 wt% based on the total weight of the pharmaceutical composition, preferably in a range of 47 to 78 wt% based on the total weight of the pharmaceutical composition, more preferably in a range of 52 to 77 wt% based on the total weight of the pharmaceutical composition and most preferably in a range of 60 to 75 wt% based on the total weight of the pharmaceutical composition.

In view of an improved stability, dissolution and rapid disintegration as well as good content uniformity, in a preferred embodiment of the present invention the immediate-release oral pharmaceutical composition is an immediate-release tablet comprising a fixed-dose combination of ivermectin and albendazole, wherein said immediate-release tablet comprises at least one excipient selected from the group of mannitol and/or croscarmellose sodium, as filler and/or disintegrant, in a range of 52 to 77 wt% based on the total weight of the pharmaceutical composition and most preferably in a range of 60 to 75 wt% based on the total weight of the pharmaceutical composition and an amount of the above-mentioned total degradation products of not more than 1.0% by weight, more preferably of not more than 0.5% by weight based on the total weight of the pharmaceutical composition after storage for 12 months at 25°C/60% RH or after storage for 6 months at 40°C/75% RH.

In a more preferred embodiment the immediate-release pharmaceutical composition is an immediate-release tablet comprising a fixed-dose combination of ivermectin and albendazole as the benzimidazole carbamate, wherein said immediate-release tablet comprises at least one excipient selected from the group of mannitol and/or croscarmellose sodium, wherein the total amount of mannitol and/or croscarmellose sodium is in a range of 37 to 88 wt% based on the total weight of the pharmaceutical composition, preferably in a range of 47 to 78 wt% based on the total weight of the pharmaceutical composition, more preferably in a range of 52 to 77 wt% based on the total weight of the pharmaceutical composition and most preferably in a range of 60 to 75 wt% based on the total weight of the pharmaceutical composition, wherein the amount of ivermectin present in said immediate-release tablet is 5 to 21 mg either as ivermectin or as a pharmaceutically acceptable salt or solvate thereof, more preferably 7 to 20 mg, more preferably 8 to 19 mg, even more preferably 9 to 18 mg, most preferably 9 or 18 mg, and wherein the amount of albendazole present in said immediate-release tablet is 200 to 800 mg either as albendazole or as a pharmaceutically acceptable salt or solvate thereof, more preferably 250 to 600 mg, even more preferably 350 to 450 mg, most preferably 400 mg.

In one embodiment of the present invention the pharmaceutical composition is an immediate-release tablet comprising a fixed-dose combination of ivermectin and albendazole comprising at least one excipient selected from the group of mannitol and/or croscarmellose sodium, wherein the total amount of mannitol and/or croscarmellose sodium is in the range of 52 to 77 wt% based on the total weight of the pharmaceutical composition, and most preferably in a range of 60 to 75 wt% based on the total weight of the pharmaceutical composition, ivermectin in an amount of 8 to 19 mg, even more preferably 9 to 18 mg, most preferably 9 or 18 mg and albendazole in in an amount of 350 to 450 mg, most preferably 400 mg.

In preferred embodiments of the present invention the pharmaceutical compositions in the form of an oral immediate-release tablet discussed above comprise both mannitol and croscarmellose in the above defined amount ranges, wherein the amount of croscarmellose is in a range of 4% to less than 25% by weight based on the total weight of the pharmaceutical composition, more preferably in a range of 5.5% to less than 15% based on the total weight of the pharmaceutical composition, because it was found according to the present invention that pharmaceutical compositions comprising such an amount of croscarmellose show in addition to an excellent stability also an improved dissolution profile of albendazole as the benzimidazole carbamate.

In an especially preferred embodiment of the present invention the pharmaceutical compositions in the form of an oral immediate-release tablet discussed above may be present as oro-dispersible immediate-release tablets.

In a particularly preferred embodiment of the present invention the content uniformity of ivermectin in the pharmaceutical composition according to the present invention is within a pharmaceutically acceptable range, preferably provides an acceptance value (AV) of not more than 15, more preferably of not more than 14, more preferably of not more than 13, even more preferably of not more than 12, even more preferably of not more than 11, even more preferably of not more than 10, still more preferably of not more than 9, still more preferably of not more than 8, particularly more preferably of not more than 7, even more preferably of not more than 6, still more preferably of not more than 5, especially more preferably of not more than 4, wherein the acceptance value (AV) is selected from the following formulae based on the media/mean value of the determinations/samples:
- If 98.5%≤ media/mean value ≤ 101.5% then: AV = K·s
- If media/mean value ≤ 98.5% then: AV = 98.5 - Media/Mean value + K·s
- If media/mean value > 101.5% then: AV = Media/Mean value - 101.5 + K·s
wherein
K represents the acceptability constant which is 2.4 (for a number of determinations/samples n = 10),
s represents the sample standard deviation (SD), and
M represents:

| | | | |
|---|---|---|---|
| M to be applied when T ≦101.5 | Reference value | If 98.5% ≤ x̅ ≤ 101.5%, then | M = x̅ (AV = Ks) |
| | | If x̅ < 98.5%, then | M = 98.5% (AV = 98.5 - x̅ + Ks) |
| | | If x̅ > 101.5%, then | M = 101.5% (AV= x̅ - 101.5 + Ks) |

For all embodiments of the first aspect of the present invention it is particularly preferred that the water content of the pharmaceutical composition comprising a fixed-dose combination of ivermectin and a benzimidazole carbamate, preferably the water content of an immediate release tablet comprising a fixed-dose combination of ivermectin and albendazole as a benzimidazole carbamate is ≤ 2% by weight, preferably ≤ 1% by weight based on the total weight of the pharmaceutical composition.

In a second aspect the present invention provides a method of producing an oral pharmaceutical composition in the form of a fixed-dose combination comprising ivermectin, a benzimidazole carbamate and at least one pharmaceutically acceptable excipient, wherein said oral pharmaceutical composition comprises ivermectin or a pharmaceutically acceptable salt or solvate thereof in an amount of 5 to 25 mg.

With respect to the oral pharmaceutical composition in the form of a fixed-dose combination, said oral pharmaceutical composition in the form of a fixed-dose combination can be produced by a process comprising the following steps:
- Granulating a mixture of benzimidazole carbamate with at least a part of the binder, if present, and at least a part of the amount of disintegrant, if present;
- sieving the wet mass obtained in the granulation step;
- separately adding ivermectin and the remaining part of the binder, if present, other excipients, if present, and the remaining part of the amount of disintegrant, if present, into a blender;
- mixing ivermectin and the other components to form a blend_{IVM};
- optionally sieving the blend_{IVM} comprising ivermectin by using a mesh of between 0.250 mm and 0.850 mm;
- blending the sieved composition comprising the benzimidazole carbamate, with the optionally sieved blend_{IVM} comprising ivermectin and the remaining excipients to be used;
- lubricating the mixture obtained in the blend step; and
- granulating or compressing the lubricated mixture to produce a final dosage form.

In a preferred embodiment the blend (= blend_{IVM}) comprising ivermectin and the remaining excipients is prepared by the following steps:
The remaining excipients (e.g. mango flavour, mannitol, sodium croscarmellose and optionally citric acid), ivermectin and optionally butyl hydroxyanisol are introduced into a blender. In one embodiment ivermectin and optionally butyl hydroxyanisol are added to the remaining excipients. The components are mixed. Preferably mixing is carried out at approx. 10 rpm for 3 minutes or equivalent. Once mixed, the blend is sieved 1 to 4, preferably 2 to 3, more preferably 2 times through a mesh of between 0.250 mm and 0.850 mm, preferably 0.350 mm to 0.650 mm, more preferably 0.400 mm to 0.500 mm and most preferably 0.457 mm to obtain the blend_{IVM}. This preparation method of blend_{IVM} was found to provide an excellent content uniformity of ivermectin and the other excipients, in particular of ivermectin and butyl hydroxyanisol, if present.

In a preferred embodiment according to the second aspect of the present invention the granulation step is a wet granulation step. Preferably, the wet granulation step involves a dry mixing followed by an addition of a binder solution (for example ethanol as solvent and povidone as binder) and a kneading step.

The wet granulation step preferably is carried out by using an impeller speed of more than 120 rpm, preferably of more than 150 rpm, more preferably of 180 rpm to 300 rpm and a chopper speed of more than 300 rpm, preferably of 400 to 2000 rpm so that the ingredients can be uniformly distributed.

Furthermore, it is preferred that a drying step is performed after the first granulation step and prior to the sieving step. It is particularly preferred that the drying step is performed in such a manner that the residual water content of the granulation mixture after drying is less than 2 % by weight, preferably less than 1% by weight based on the weight of the dried mixture. A fluid bed dryer can be used for drying the wet mass in the drying step.

In some embodiments the oral pharmaceutical composition in the form of a fixed-dose combination may be coated with a coating agent, such as ethylcellulose, methyl hydroxyethyl cellulose, povidone, gelatin, hydroxypropyl cellulose, hypromellose, cellulose acetate phthalate, an acrylate polymer or hydroxymethyl propyl cellulose.

In some embodiments when the oral pharmaceutical composition is a fixed-dose immediate-release tablet comprising a fixed-dose combination of ivermectin and albendazole as a benzimidazole carbamate. In a preferred embodiment such immediate-release tablets can be produced in the form of scored tablets.

According to a third aspect the present invention provides a method of treating subjects suffering from a parasitic infection, preferably soil-transmitted helminths by administering an oral pharmaceutical composition, preferably an oral immediate-release pharmaceutical composition in the form of a fixed-dose combination comprising ivermectin in an amount of 5 to 25 mg ivermectin or a pharmaceutically acceptable salt or solvate thereof, preferably 5 to 21 mg ivermectin or a pharmaceutically acceptable salt or solvate thereof, more preferably 7 to 20 mg, more preferably 8 to 19 mg, even more preferably 9 to 18 mg, and a benzimidazole carbamate in an amount of 200 to 800 mg or a pharmaceutically acceptable salt or solvate thereof, more preferably 250 to 600 mg, even more preferably 350 to 450 mg, most preferably 400 mg in at least one pharmaceutically acceptable excipient.

In one embodiment of the method of treating subjects suffering from a parasitic infection an oral pharmaceutical composition in the form of a fixed-dose combination comprising ivermectin and a benzimidazole carbamate may be administered once, twice or three times per day, preferably once per day.

In one embodiment of the method of treating subjects suffering from a parasitic infection an oral pharmaceutical composition in the form of a fixed-dose combination comprising ivermectin and a benzimidazole carbamate may be administered daily on 1 to 7 consecutive days, preferably 1 to 3 consecutive days or most preferably one day or three consecutive days.

In a particularly preferred embodiment of the method of treating subjects suffering from a parasitic infection an oral pharmaceutical composition in the form of a fixed-dose combination comprising ivermectin and a benzimidazole carbamate may be administered once daily on 1 to 3 consecutive days or most preferably once on 1 day or on 3 consecutive days.

According to the present invention the subject to be treated can be a human or an animal in the need of treatment due to a parasitic infection or at high risk of a parasitic infection, for example of soil-transmitted helminths. In a preferred embodiment the subject to be treated is a human.

The human can be an adult which is a human of an age of 18 years or older or a child which is a young person of an age of 12 months to less than 18 years. In a further preferred embodiment the child is a young person of an age of 5 years to less than 18 years.

In a particularly preferred embodiment of the present invention in the method of treating an adult human suffering from or at high risk for a parasitic infection, for example a parasitic infection caused by soil-transmitted helminths, by administering an oral pharmaceutical composition in the form of a fixed-dose combination comprising ivermectin and albendazole, wherein the pharmaceutical composition comprises ivermectin in an amount of 18 mg and albendazole in an amount of 400 mg, said pharmaceutical composition is administered once daily on 1 to 3 consecutive days or most preferably for 1 or 3 consecutive days, preferably as a single dose on one day.

In a particularly preferred embodiment of the present invention in the method of treating a human child which has a body weight of more than 45 kg suffering from or at high risk for a parasitic infection, for example a parasitic infection caused by soil-transmitted helminths, by administering an oral pharmaceutical composition in the form of a fixed-dose combination comprising ivermectin and albendazole, wherein the pharmaceutical composition comprises ivermectin in an amount of 18 mg and albendazole in an amount of 400 mg, said pharmaceutical composition is administered once daily on 1 to 3 consecutive days or most preferably 1 or 3 consecutive days, preferably as a single dose on one day.

In a particularly preferred embodiment of the present invention in the method of treating a human child which has a body weight of 45 kg or less suffering from or at high risk for a parasitic infection, for example a parasitic infection caused by soil-transmitted helminths, by administering an oral pharmaceutical composition in the form of a fixed-dose combination of ivermectin and albendazole, wherein the pharmaceutical composition comprises ivermectin in an amount of 9 mg and a benzimidazole carbamate i.e. albendazole in an amount of 400 mg, said pharmaceutical composition is administered once daily on 1 to 3 consecutive days or most preferably 1 or 3 consecutive days, preferably as a single dose on one day.

In all embodiments according to the third aspect of the present invention discussed above, the oral pharmaceutical composition in the form of a fixed-dose combination comprising ivermectin and albendazole is preferably formulated as an immediate-release tablet, most preferably formulated as an oro-dispersible immediate-release tablet.

In a further embodiment of said the oral pharmaceutical composition in the form of a fixed-dose combination comprising ivermectin and albendazole for use in the treatment of subjects suffering from a parasitic infection, for example a parasitic infection caused by soil-transmitted helminths, said oral pharmaceutical composition may be administered together (simultaneously or in any appropriate sequence) with one or more of the following additional drugs:
- Mebendazole
- Nitazoxanide
- Niclosamide
- Rafoxanide
- Pentamidine
- Praziquantel
- Resorantel
- Pyrantel
- Closantel
- Nitoxynil
- Thiabendazole
- Triclabendazole
- Trichlorfon
- Derquantel
- Monepantel
- Moxidectin
- Levamizole
- Doramectin
- Abamectin
- Oxfendazole

In a preferred embodiment the subject is suffering from at least one parasitic infection caused by soil-transmitted helminths selected from the group consisting of *Ascaris lumbricoides*; *Trichuris trichiura*; *Necator americanus, Ancylostoma duodenale* and *S*. *stercoralis.*

This invention will be better understood by reference to the non-limiting examples that follow, but those skilled in the art will readily appreciate that the examples are only illustrative of the invention and the claims which follow thereafter.

Unless otherwise indicated, all percentages of the ingredients in the present application are percentages by weight (w/w).

### EXAMPLES:

### EXAMPLE 1

### A. Preparation of Tablets

By using the components listed below, several immediate-release tablets were manufactured according to the following process.

A binding solution is prepared by adding anhydrous ethanol and povidone into a solution tank and stirred until complete dissolution.

Thereafter, albendazole and 50% by weight of the amount of sodium croscarmellose mentioned below are mixed in a granulator. Binding solution is then sprayed until granulates are obtained while mixing the mixture. The granules obtained are loaded into a fluid bed and dried at temperatures of up to 50 °C until the granules have a water content of ≤1.0% by weight (Karl Fischer analysis). After sieving the granules, the granules are mixed with a separately prepared blend_{IVM} (see below) comprising ivermectin, mango flavour, mannitol, the remaining 50% by weight of sodium croscarmellose and optionally anhydrous citric acid and/or butyl hydroxyanisole. Thereafter, the blend obtained is mixed with sodium stearyl fumarate. Then the mixture is compressed by using a commercial tablet press to obtain tablets having an average weight of 1200.00 mg.

### Preparation of the blend_{IVM}

The blend_{IVM} is prepared by adding mango flavour, mannitol, sodium croscarmellose and optionally citric acid into a blender, thereafter ivermectin and optionally butyl hydroxyanisol are added. The components are mixed at approx. 10 rpm for 3 minutes or equivalent. Once mixed, the blend is sieved twice through a mesh 0.457 mm to obtain the blend_{IVM}.

### Composition 1A

| **FUNCTION** | **MATERIAL** | **AMOUNT [mg]** | **%** |
|---|---|---|---|
| ACTIVE INGREDIENT | ALBENDAZOLE | 400,00 mg | 33,33% |
| ACTIVE INGREDIENT | IVERMECTIN | 18,00 mg | 1,50% |
| FILLER | MANNITOL | 595,00 mg | 49,59% |
| | CROSPOVIDONE | 36,00 mg | 3,00% |
| | POLYVINYL ACETATE | 34,00 mg | 2,83% |
| | POVIDONE | 3,00 mg | 0,25% |
| SWEETENING AGENT | SUCRALOSE | 6,000 mg | 0,50% |
| DISINTEGRANT | CROSCARM ELLOSE SODIUM | 60,00 mg | 5,00% |
| BINDER | POVIDONE K-30 | 12,00 mg | 1,00% |
| FLAVOR | MANGO FLAVOUR | 24,00 mg | 2,00% |
| LUBRICANT | SODIUM STEARYL FUMARATE | 12,00 mg | 1,00% |
| | **TOTAL** | **1200,00 mg** | **100,00%** |

### Composition 2A

| **FUNCTION** | **MATERIAL** | **AMOUNT [mg]** | **%** |
|---|---|---|---|
| ACTIVE INGREDIENT | ALBENDAZOLE | 400,00 mg | 33,33% |
| ACTIVE INGREDIENT | IVERMECTIN | 18,00 mg | 1,50% |
| FILLER | MANNITOL | 627,06 mg | 52,25% |
| | CROSCARMELLOSE SODIUM | 33,00 mg | 2,75% |
| ANTIOXIDANT | BUTYL HYDROXYANISOLE | 0,144 mg | 0,012% |
| SEQUESTERING AGENT | CITRIC ACID ANHYDROUS | 1,80 mg | 0,15% |
| DISINTEGRANT | CROSCARMELLOSE SODIUM | 60,00 mg | 5,00% |
| BINDER | POVIDONE K-30 | 12,00 mg | 1,00% |
| FLAVOR | MANGO FLAVOUR | 24,00 mg | 2,00% |
| LUBRICANT | SODIUM STEARYL FUMARATE | 24,00 mg | 2,00% |
| | **TOTAL** | **1200,00 mg** | **100,00%** |

### Composition 2B

| **FUNCION** | **MATERIAL** | **AMOUNT [mg]** | **%** |
|---|---|---|---|
| ACTIVE INGREDIENT | ALBENDAZOLE | 400,00 mg | 33,33% |
| ACTIVE INGREDIENT | IVERMECTIN | 9,00 mg | 0,75% |
| FILLER | MANNITOL | 635,61 mg | 52,96% |
| | CROSCARMELLOSE SODIUM | 33,45 mg | 2,79% |
| ANTIOXIDANT | BUTYLHYDROXYANISOLE | 0,14 mg | 0,01% |
| SEQUESTERING AGENT | CITRIC ACID ANHYDROUS | 1,80 mg | 0,15% |
| DISINTEGRANT | CROSCARM ELLOSE SODIUM | 60,00 mg | 5,00% |
| BINDER | POVIDONE K-30 | 12,00 mg | 1,00% |
| FLAVOR | MANGO FLAVOUR | 24,00 mg | 2,00% |
| LUBRICANT | SODIUM STEARYL FUMARATE | 24,00 mg | 2,00% |
| | **TOTAL** | **1200,00 mg** | **100,00%** |

### B. Stability Testing

The tablet compositions outlined above were subjected to a stability testing under storage conditions of 25 °C/60% relative humidity and 40 °C/75% relative humidity, respectively.

The results are shown in the tables below:

### STABILITY REPORT COMPOSITION 1A

| **Product Name/ Strength** | Ivermectin/Albendazole tablets 18/400mg | **Active Pharmaceutical Ingredient** | | Ivermectin /Albendazole | |
|---|---|---|---|---|---|
| **Storage condition** | 25°C/60%RH | | | | |

| **Test** | **Specification** | **Initial** | **Time in Months** | | |
|---|---|---|---|---|---|
| | | | **1** | **2** | **4** |
| **Description** | Round, white-almost white, round tablets of approximately 16mm of diameter | Conform | Conform | Conform | Not conform |
| **Assay Ivermectin** | 95.0%-105.0% | 98.6% | 98.4% | 95.2% | 93.3% |
| **Assay Albendazole** | 95.0%-105.0% | 100.7 | 96.8% | 102.3% | 99.7% |
| **Water content (KF)** | NMT 2.0% | 1.0% | 1.0% | 0.9% | 0.7% |
| **Dissolution Ivermectin** | NLT 80% (Q) at 45min | 88% | 90% | 89% | 91% |
| **Dissolution Albendazole** | NLT 80% (Q) at 45min | 93% | 92% | 92% | 92% |
| **Related Substances Ivermectin** | Hydroxyoxy impurity (NMT 0.5%) | NR | NR | NR | 0.6% |
| | Any unknown impurity (NMT 0.2%) | NR | NR | NR | NR |
| | | NR | NR | NR | 0.6% |
| | Total impurities (NMT 5%) | | | | |
| **Related Substances Albendazole** | Unknown maximum individual impurity (NMT 0.2%) | NR | NR | NR | NR |
| | Total degradation products (NMT 1.3%) | NR | NR | NR | NR |

| | | | | | |
|---|---|---|---|---|---|
| ND: Not detected / NR: Not reportable NMT: Not more than / NLT: Not less than | | | | | |

### STABILITY REPORT COMPOSITION 1A

| **Product Name/ Strength** | Ivermectin/Albendazole tablets 18/400mg | **Active Pharmaceutical Ingredient** | | Ivermectin /Albendazole | |
|---|---|---|---|---|---|
| **Storage condition** | 40°C/75%RH | | | | |

| **Test** | **Specification** | **Initial** | **Time in Months** | | |
|---|---|---|---|---|---|
| | | | **1** | **2** | **4** |
| **Description** | Round, white-almost white, round tablets of approximately 16mm of diameter | Conform | Not Conform | Not Conform | Not Conform |
| **Assay Ivermectin** | 95.0%-105.0% | 98.6% | 98.3% | 94.9% | 87.5% |
| **Assay Albendazole** | 95.0%-105.0% | 100.7 | 96.1% | 101.8% | 100.1% |
| **Water content (KF)** | NMT 2.0% | 1.0% | 0.6% | 0.9% | 0.7% |
| **Dissolution Ivermectin** | NLT 80% (Q) at 45min | 88% | 89% | 89% | 87% |
| **Dissolution Albendazole** | NLT 80% (Q) at 45min | 93% | 86% | 83% | 92% |
| **Related Substances Ivermectin** | Hydroxyoxy impurity (NMT 0.5%) | NR | NR | 1.0% | 1.1% |
| | Any unknown impurity (NMT 0.2%) | NR | NR | NR | NR |
| | | NR | NR | 1.0% | 1.1% |
| | Total impurities (NMT 5%) | | | | |
| **Related Substances Albendazole** | Unknown maximum individual impurity (NMT 0.2%) | NR | NR | NR | NR |
| | Total degradation products (NMT 1.3%) | NR | NR | NR | NR |

| | | | | | |
|---|---|---|---|---|---|
| ND: Not detected / NR: Not reportable NMT: Not more than / NLT: Not less than | | | | | |

### STABILITY REPORT COMPOSITION 2A

| **Product Name/ Strength** | Ivermectin/Albendazole tablets 18/400mg | | **Active Pharmaceutical Ingredient** | | Ivermectin /Albendazole | |
|---|---|---|---|---|---|---|
| **Storage condition** | 25°C/60%RH | | | | | |

| **Test** | **Specification** | **Intial** | **Time in Months** | | | |
|---|---|---|---|---|---|---|
| | | | **3** | **6** | **9M** | **12M** |
| **Description** | Round, white-almost white, round tablets of approximately 16mm of diameter | Conform | Conform | Conform | Conform | Conform |
| **Assay Ivermectin** | 95.0%-105.0% | 99,6% | 100,6% | 97,8% | 99,8% | 98,3% |
| **Assay Albendazole** | 95.0%-105.0% | 102,3% | 99,7% | 98,5% | 98,8% | 98,1% |
| **Water content (KF)** | NMT 2.0% | 0,9% | 0,4% | 0,3% | 0,7% | 0,5% |
| **Dissolution Ivermectin** | NLT 80% (Q) at 45min | 930% | 93% | 93% | 93% | 93% |
| **Dissolution Albendazole** | NLT 80% (Q) at 45min | 86% | 95% | 97% | 99% | 98% |
| **Related Substances Ivermectin** | Hydroxyoxy impurity (NMT 0.5%) | 0,2 | 0,2 | 0,2 | 0,2 | 0,2 |
| | Any unknown impurity (NMT 0.2%) | NR | NR | NR | NR | NR |
| | | 0,2 | 0,2 | 0,2 | 0,2 | 0,2 |
| | Total impurities (NMT 5%) | | | | | |
| **Related Substances Albendazole** | Unknown maximum individual impurity (NMT 0.2%) | NR | NR | NR | NR | NR |
| | Total degradation products (NMT 1.3%) | NR | NR | NR | NR | NR |

| | | | | | | |
|---|---|---|---|---|---|---|
| ND: Not detected / NR: Not reportable NMT: Not more than / NLT: Not less than | | | | | | |

### STABILITY REPORT COMPOSITION 2A

| **Product Name/ Strength** | Ivermectin/Albendazole tablets 18/400mg | **Active Pharmaceutical Ingredient** | | Ivermectin /Albendazole | |
|---|---|---|---|---|---|
| **Storage condition** | 40°C/75%RH | | | | |

| **Test** | **Specificat** | **Initial** | **Time in Months** | |
|---|---|---|---|---|
| | | | **3** | **6** |
| **Description** | Round, white-almost white, round tablets of approximately 16mm of diameter | Conform | Conform | Conform |
| **Assay Ivermectin** | 95.0%-105.0% | 99,6% | 100,0% | 96,1% |
| **Assay Albendazole** | 95.0%-105.0% | 102,3% | 99,1% | 98,4% |
| **Water content (KF)** | NMT 2.0% | 0,9% | 0,2% | 0,3% |
| **Dissolution Ivermectin** | NLT 80% (Q) at 45min | 93% | 95% | 93% |
| **Dissolution Albendazole** | NLT 80% (Q) at 45min | 86% | 94% | 92% |
| | Hydroxyoxy impurity (NMT 0.5%) | 0,2 | 0,2 | 0,2 |
| **Related Substances Ivermectin** | Any unknown impurity (NMT 0.2%) | NR | NR | NR |
| | Total impurities (NMT 5%) | 0,2 | 0,2 | 0,2 |
| **Related Substances Albendazole** | Unknown maximum individual impurity (NMT 0.2%) | NR | NR | NR |
| | Total degradation products (NMT 1.3%) | NR | NR | NR |

### C. Determination of dissolution profiles

The following two tablet compositions were prepared according to the wet granulation process described above.

Thereafter, the dissolution profile of ivermectin in these compositions was tested in 900 ml phosphate buffer, pH 7.0 + 0.5% w/v sodium lauryl sulphate with a USP device II (paddles) at 50 rpm.

The dissolution profile of albendazole in these compositions was tested in 900 ml 0.1 N HCl, pH 1.2 + 1% w/v sodium lauryl sulphate with a USP device II (paddles) at 50 rpm.

**Tablet Composition 3(BN 069)**

| **FUNCION** | **MATERIAL** | **FORMULA UNITARIA** | **%** |
|---|---|---|---|
| ACTIVE INGREDIENT | ALBENDAZOLE | 400,00 mg | 33,33% |
| ACTIVE INGREDIENT | IVERMECTIN | 9,00 mg | 0,75% |
| FILLER | Mannitol | 635,61 mg | 52,96% |
| | CROSCARMELLOSE SODIUM | 33,45 mg | 2,79% |
| ANTIOXIDANT | BUTYLHYDROXYANISOLE | 0,14 mg | 0,01% |
| SEQUESTERING AGENT | CITRIC ACID ANHYDROUS | 1,80 mg | 0,15% |
| DISINTEGRANT | CROSCARM ELLOSE SODIUM | 60,00 mg | 5,00% |
| BINDER | POVIDONE K-30 | 12,00 mg | 1,00% |
| FLAVOR | MANGO FLAVOUR | 24,00 mg | 2,00% |
| LUBRICANT | SODIUM STEARYL FUMARATE | 24,00 mg | 2,00% |
| | **TOTAL** | **1200,00 mg** | **100,00%** |

**Tablet Composition 4 (BN 062)**

| **FUNCION** | **MATERIAL** | **FORMULA UNITARIA** | **%** |
|---|---|---|---|
| ACTIVE INGREDIENT | ALBENDAZOLE | 400,00 mg | 33,33% |
| ACTIVE INGREDIENT | IVERMECTIN | 9,00 mg | 0,75% |
| FILLER | Mannitol | 635,61 mg | 57,71% |
| | CROSCARMELLOSE SODIUM | 33,45 mg | 3,04% |
| ANTIOXIDANT | BUTYLHYDROXYANISOLE | 0,14 mg | 0,01% |
| SEQUESTERING AGENT | CITRIC ACID ANHYDROUS | 1,80 mg | 0,15% |
| DISINTEGRANT | CROSCARM ELLOSE SODIUM | 0 mg | 0,00% |
| BINDER | POVIDONE K-30 | 12,00 mg | 1,00% |
| FLAVOR | MANGO FLAVOUR | 24,00 mg | 2,00% |
| LUBRICANT | SODIUM STEARYL FUMARATE | 24,00 mg | 2,00% |
| | **TOTAL** | **1200,00 mg** | **100,00%** |

### Ivermectin Dissolution Profile:

| Time (min) | Ivermectin/Albendazole 9/400 mg tablets BN:069 | | Ivermectin/Albendazole 9/400 mg tablets BN:062 | |
|---|---|---|---|---|
| | Average (%) | RSD (%) | Average (%) | RSD (%) |
| 0 | 0 | 0 | 0 | 0 |
| 5 | 58 | 6 | 69 | 2 |
| 10 | 85 | 2 | 79 | 2 |
| 15 | 88 | 2 | 81 | 2 |
| 20 | 88 | 1 | 81 | 2 |
| 30 | 88 | 1 | 81 | 2 |
| 45 | 88 | 1 | 82 | 3 |

### Albendazole Dissolution Profile:

| Time (min) | Ivermectin/Albendazole 9/400 mg tablets BN:069 | | Ivermectin/Albendazole 9/400 mg tablets BN:062 | |
|---|---|---|---|---|
| | Average (%) | RSD (%) | Average (%) | RSD (%) |
| 0 | 0 | 0 | 0 | 0 |
| 5 | 85 | 3 | 21 | 5 |
| 10 | 94 | 3 | 25 | 4 |
| 15 | 95 | 3 | 26 | 4 |
| 20 | 93 | 4 | 27 | 4 |
| 30 | 93 | 3 | 28 | 3 |
| 45 | 92 | 2 | 31 | 3 |

### D. Determination of Content Uniformity

### PROCESS:

Tablets of Composition 2A and tablets of Composition 2B were prepared according to the method disclosed in Example 1 above. Tablets of a Comparative Composition I were prepared following the same method steps, except that the 0.457 mm mesh used for sieving Compositions 2A and 2B was replaced by a 1.00 mm mesh for preparation of tablets of Comparative Composition I. Each blend was sieved twice through the mesh in a process for preparing tablets of Compositions 2A, 2B and once for preparing tablets of a Comparative Composition I.

In the following the acceptance value (AV) is selected from the following formulae based on the media/mean value of the determinations/samples:
- If 98.5%≤ media/mean value ≤ 101.5% then: AV = K·s
- If media/mean value ≤ 98.5% then: AV = 98.5 - Media/Mean value + K·s
- If media/mean value > 101.5% then: AV = Media/Mean value - 101.5 + K·s
wherein
K represents the acceptability constant which is 2.4 (for a number of determinations/samples n = 10),
s represents the sample standard deviation (SD), and
M represents:

| | | | |
|---|---|---|---|
| M to be applied when T ≦101.5 | Reference value | If 98.5% ≤ x̅ ≤ 101.5%, then | M = x̅ (AV = Ks) |
| | | If x̅ < 98.5%, then | M = 98.5% (AV = 98.5 - x̅ + Ks) |
| | | If x̅ > 101.5%, then | M = 101.5% (AV = x̅ - 101.5 + Ks) |

### CONTENT UNIFORMITY BHA:

### SPECIFICATION BHA UNIFORMITY OF DOSAGE UNITS (CONTENT UNIFORMITY): AV ≤ 15.0

### Blend sieved twice through mesh 0.457 mm

| **Blend** | **Analysis Content Uniformity** | **Sample** | **Result %** | **Media/Mean value (%)** | **RSD%** | **SD** | **Acceptance Value AV** |
|---|---|---|---|---|---|---|---|
| Composition 2B | BHA | 1 | 100,5 | 104,0 | 2,3 | 2,4 | 8,2 |
| | | 2 | 103,9 | | | | |
| | | 3 | 100,9 | | | | |
| | | 4 | 106,9 | | | | |
| | | 5 | 107,6 | | | | |
| | | 6 | 103,6 | | | | |
| | | 7 | 104,6 | | | | |
| | | 8 | 106,1 | | | | |
| | | 9 | 102,8 | | | | |
| | | 10 | 103,4 | | | | |

### Blend sieved once through mesh 1.00mm

| **Blend** | **Analysis Content Uniformity** | **Sample** | **Result %** | **Media/Mean value(%)** | **RSD%** | **SD** | **Acceptance Value AV** |
|---|---|---|---|---|---|---|---|
| Comparative Composition I | BHA | 1 | 106,3 | 113,1 | 19,0 | 21,5 | 63,3 |
| | | 2 | 130,7 | | | | |
| | | 3 | 104,4 | | | | |
| | | 4 | 84,5 | | | | |
| | | 5 | 103,8 | | | | |
| | | 6 | 116,5 | | | | |
| | | 7 | 80,7 | | | | |
| | | 8 | 128,8 | | | | |
| | | 9 | 125,5 | | | | |
| | | 10 | 150,2 | | | | |

Only composition 2B and Composition 2A (which also met the criteria of AV<15) were shown to have a high content uniformity of BHA, whereas Comparative Composition I showed an unacceptable low content uniformity of BHA.

### CONTENT UNIFORMITY IVM

### SPECIFICATION IVERMECTIN UNIFORMITY OF DOSAGE UNITS (CONTENT UNIFORMITY): AV ≤ 15.0

### Blend sieved twice through mesh 0.457 mm

| **Blend** | **Analysis Content Uniformity** | **Sample** | **Result %** | **Media/Mean value (%)** | **RSD%** | **SD** | **Acceptance Value (AV)** |
|---|---|---|---|---|---|---|---|
| Composition 2A | IVM | 1 | 102,1 | 100,5 | 1,9 | 1,9 | 4,7 |
| | | 2 | 101,1 | | | | |
| | | 3 | 101,1 | | | | |
| | | 4 | 97,2 | | | | |
| | | 5 | 103,5 | | | | |
| | | 6 | 101,8 | | | | |
| | | 7 | 98,5 | | | | |
| | | 8 | 101,4 | | | | |
| | | 9 | 99,9 | | | | |
| | | 10 | 98,4 | | | | |

### Blend sieved twice through mesh 0.457 mm

| **Blend** | **Analysis Content Uniformity** | **Sample** | **Result %** | **Media/Mean value (%)** | **RSD%** | **SD** | **Acceptance Value (AV)** |
|---|---|---|---|---|---|---|---|
| Composition 2B | IVM | 1 | 101,9 | 100,1 | 1,2 | 1,2 | **2,8** |
| | | 2 | 101,7 | | | | |
| | | 3 | 100,5 | | | | |
| | | 4 | 101,1 | | | | |
| | | 5 | 98,5 | | | | |
| | | 6 | 98,6 | | | | |
| | | 7 | 99,5 | | | | |
| | | 8 | 99,7 | | | | |
| | | 9 | 99,9 | | | | |
| | | 10 | 99,6 | | | | |

Only composition 2B and Composition 2A were shown to have a high content uniformity of IVM, whereas Comparative Composition I showed an unacceptable low content uniformity of BHA not meeting the AV of <15.

### EXAMPLE 2

### First clinical trial

A clinical trial was conducted in Kenya as a unicentric, 3-arm, parallel, open-label, individually randomized trial that determined in three weight groups the safety of the FDC (fixed dose co-formulation) given as a single day dose (FDC) or 3-day single dose regimen (FDC x3) for the treatment of Soil-Transmitted Helminth Infections (Trichuris trichiura, hookworm, Strongyloides stercoralis). The study was performed in children and young adult.

Efficacy was assessed by analysis of stool samples at Day 21 (±7 day) post-treatment in order to determine CR for each individual STH species. The analysis methods were Kato Katz for T. trichiura and hookworms, and Baermann for S. stercoralis [Anderson 2014; Verweij 2014]. Cure is defined as absence of the species of STH in participants who had a positive egg count and/or larva for that STH at baseline. CR is defined as the proportion of individuals cured (absence of any egg) to the total of those infected at baseline with each particular species of STH.

Efficacy was also assessed by calculating parasite burden decrease, and calculating the egg reduction rates (ERR) for T. trichiura, hookworms, and A. lumbricoides.

Regarding the Safety assessments, both components included evaluation of AEs, vital signs, physical examination, weight, height, and body mass index (BMI). The safety assessments were conducted from Day 0 to Day 3, and then on Day 7 following dose administration.

In the first clinical trial, the design of study planned a total of 126 participants with T. trichiura infection (confirmed by Kato-Katz technique in a fresh stool sample) to be enrolled in a sequential manner per weight group to administer the desired dose, starting from 300-391 µg/kg ivermectin (IVM). Participants were stratified in different weight groups per study design: Group 1 (23-30 kg) 38 participants, group 2 (30-45 kg) 38 participants and group 3 (15-23 kg) 50 participants in order to gradually increase the dose of IVM in the FDC. Then, participants were allocated by simple randomization to one of the three study arms:
- Treatment Arm 1: Albendazole (ALB) 400 mg as a single daily dose
- Treatment Arm 2: IVM/ALB FDC (9 mg/400 mg or 18 mg/400 mg based on weight group) as a single daily dose.
- Treatment Arm 3: IVM/ALB FDC (9 mg/400 mg or 18 mg/400 mg based on weight group) as a single daily dose for three days.

Overall, 135 study participants were randomized to one of the 3 treatment arms and 128 (94.8%) received study drug. The exposure to the planned study drug administration was > 90% across the 3 study arms. In Group 1 and Group 3, all participants in the FDCx1 (n=35) and FDCx3 (n=36) arms received the FDC dose of 9 mg/400 mg. Whereas in Group 3, all FDCx1 (n=15) and FDCx3 (n=15) participants received the FDC dose of 18 mg/400 mg.

The proportions of males and females were balanced (58.5% males and 41.5% females).

There were no significant differences in the demographic characteristics of the participants in the FDC Single Dose or FDC x3 arms compared with the single dose ALB arm, including age, age category, sex, weight group, and BMI.

Overall, reports of photophobia, blurred vision, visual impairment, pruritus, and skin lesions compatible with scabies were infrequent (< 2% each) and there were no differences in incidences between the FDC treatment arms compared with the ALB arm (p > 0.05 or not evaluable for each comparison).

All participants (100%) were positive for *T. trichiura.*

The main objective of this clinical trial was the evaluation of safety for a total of 135 participants. No alterations in vital signs were observed among the participants in the three treatment groups throughout the study. A total of 27 participants (20.0% overall) experienced 35 TEAEs, including 22 participants (21.0%) in the pooled FDC arms (hereafter referred to as the FDC Pooled group) and 5 participants (16.7%) in the ALB arm. There were no severe TEAEs, no treatment-emergent SAEs, and no TEAEs leading to study drug discontinuation.

The secondary objectives were to evaluate the efficacy of FDC against *T. trichiura* in a paediatric population, and for *hoowworms* and *Strongyloides (S.) stercoralis* in participants co-infected with species concomitantly to their infections with *T. trichiura.*

The *T. trichiura* CRs by microscopy were significantly higher for the FDC Single Dose arm (72.5%) and FDC x3 arm (92.6%) compared with the ALB arm (30.0%) (p < 0.0167 for both comparisons). The ERRs for *T. trichiura* are supportive of the CR results, showing significantly higher ERR for both FDC arms compared with the ALB arm (p < 0.0001 for both comparisons).

The *T. trichiura* CR was also evaluated by qPCR, and the results were consistent with the result based on microscopy, showing significantly higher CRs for the FDC Single Dose arm (59.2%) and FDC x3 arm (83.3%) compared with the ALB arm (27.6%) (p < 0.0167 for both comparisons).

### Second clinical trial

A second clinical trial was conducted in Kenya, Ethiopia, and Mozambique as a multicentric, single-blinded, 3-arm, randomized, active-controlled, parallel-group, superiority study and determined in two weight groups and in which the efficacy of FDC single dose or FDC x3 compared with active control (i.e., ALB single dose) for the treatment of STH infections (T. trichiura, hookworm, S. stercoralis) was measured. The study was performed in children and young adult aged between 5 to 18 years.

Efficacy was assessed by analysis of stool samples at Day 21 (±7 day) post-treatment in order to determine CR for each individual STH species. The analysis methods were Kato Katz for T. trichiura and hookworms, and Baermann for S. stercoralis [Anderson 2014; Verweij 2014]. Cure is defined as absence of the species of STH in participants who had a positive egg count and/or larva for that STH at baseline. CR is defined as the proportion of individuals cured (absence of any egg) to the total of those infected at baseline with each particular species of STH.

Efficacy was also assessed by calculating parasite burden decrease, and calculating the egg reduction rates (ERR) for T. trichiura, hookworms, and A. lumbricoides.

Regarding the safety assessments, both components included evaluation of AEs, vital signs, physical examination, weight, height, and body mass index (BMI). The safety assessments were conducted from Day 0 to Day 3, and then on Day 7 following dose administration.

In the second clinical trial, the median age of participants was 11.0 years (range 5 to 18).

There were no significant differences in the demographic characteristics of age, age group, sex, weight category, and BMI for the participants in the FDC arms compared with the ALB arm (p > 0.05 for each comparison).

Overall, reports of photophobia, blurred vision, visual impairment, pruritus, and skin lesions compatible with scabies were infrequent (<1.5% each) and there were no differences in incidences between the FDC treatment arms compared with the ALB arm (p > 0.05 for each comparison).

From the total 1223 participants in the sample size for the adaptive, 1097 were planned to be enrolled in the second clinical trial. Finally, the second clinical trial portion of the study randomized 866 participants, and all (100%) received study drug. Exposure to the planned study drug administration was ≥ 98% across the 3 study arms.

The participants were assigned to 1 of 3 study arms by block randomization (*T*. *trichiura* 1:2:2; *hookworms* 1:1:1; *S*. *stercoralis* 2:5:5) and stratified by species of STH (Table 12).
- Treatment Arm 1: ALB 400 mg as a single daily dose (active control arm).
- Treatment Arm 2: IVM/ALB FDC (doses based on body weight; ≥ 45 kg: 18 mg/400 mg and < 45 kg: 9 mg/400 mg) as a single daily dose
- Treatment Arm 3: IVM/ALB FDC (doses based on body weight; ≥ 45 kg: 18 mg/400 mg and < 45 kg: 9 mg/400 mg) as a daily dose for 3 days.

Of the 866 participants who received the study drugs, 213 received ALB, 330 received FDCx1 (277 received 9 mg/400 mg, and 53 received 18 mg/400 mg) and 323 received FDCx3 (271 received 9 mg/400 mg, and 52 received 18 mg/400 mg).

The objectives in the second clinical trial component included a primary objective to evaluate the efficacy of FDC single dose and FDC x3 compared to the standard single dose regimen of ALB (400 mg) through the cure rate (CR) and Egg Reduction Rate (ERR) for the treatment of *T. trichiura* in a paediatric and young adult population 21 days post-treatment and a set of secondary objectives:
- To evaluate the efficacy of FDC as a single dose and FDC x3 for the treatment of *hookworm* and *S*. *stercoralis.*
- To evaluate the safety of FDC as a single dose and FDC x3 for the treatment of *T*. *trichiura,* hookworm and S. *stercoralis.*
- To evaluate the performance of qPCR in calculating the primary outcome measurement (efficacy) compared to an egg counting method (Kato-Katz).
- To evaluate the frequency of known ALB-resistant alleles in hookworm and *T*. *trichiura* in the 3 treatment arms before and after treatment.

All safety and efficacy analyses were based on the Intent-to-treat (ITT) population: All participants who were randomized in the study.

All the results showed in this section are collected with the first and second clinical trial participants (pooled analysis in the tables).

The primary endpoint results for the higher estimated CR for *T. trichiura* at 21 days after treatment, using microscopy, resulted to be as expected in participants allocated to Arms 2 and 3 (FDC arms) compared with Arm 1 (control, ALB). The efficacy of FDC in curing *T*. *trichiura* observed in the first clinical trial portion of the study were consistent with and supportive of the results of the pooled analyses conducted for the second clinical trial component showing significantly higher *T. trichiura* CR for the FDC x3 arm (97.2%) and for the FDC Single Dose arm (82.9%) compared with the ALB arm (35.9%; p < 0.0001 for both comparisons) (Table 1).

**Table 1. Cure Rate for Participants Infected with T. trichiura at 21 Days After Treatment by Microscopy (first and second clinical trial ITT Population)**

| | | **FDC 400 mg ALB - 18/9 mg IVM** | |
|---|---|---|---|
| | **ALB 400 mg Single Dose (N = 243)** | **Single Dose (N = 381)** | **Daily Dose x3 Days (N = 377)** |
| **First and second clinical trial Pooled** | | | |
| Number of participants positive for infection with *T*. *trichiura* at pre-treatment, n | 131 | 251 | 254 |
| Number of participants cured at post-treatment, n | 47 | 208 | 247 |
| Cure rate (CR), % (95% Cl) | 35.9 (27.7, 44.1) | 82.9 (78.2, 87.5) | 97.2 (95.2, 99.3) |
| Difference in CR (vs. ALB 400 mg Single Dose) | | | |
| Difference^{a} | - | 47.2 | 61.3 |
| P-value^{b} | - | < 0.0001 | < 0.0001 |

| | | | |
|---|---|---|---|
| Abbreviations: ALB=albendazole; CR=cure rate; FDC=fixed-dose co-formulation (ALB/IVM); ITT=intent-to-treat; IVM=ivermectin. a. Difference in cure rates, expressed in percentages, and based on Mantel Haenszel methods to account for stratification by site. b. P-values are based on the Cochran-Mantel-Haenszel test, controlling for the effect of site. Notes: A participant with multiple infections was included in the analysis of each target species that the participant was infected with. Participants missing the post-treatment stool sample were considered as not cured. Participants withdrawn before Visit 6 (21 days after treatment) with stool sample were included in the analysis. | | | |

The ERR for *T. trichiura* at 21 days after treatment, by microscopy revealed to be significantly higher for the FDC x3 arm (99.9%) and FDC Single Dose arm (99.2%) compared with the ALB arm (83.4%; p < 0.0001 for both comparisons) (Table 2).

**Table 2. The Egg Reduction Rate for T. trichiura at 21 Days After Treatment by Microscopy (first and second clinical trial ITT Population)**

| | **ALB 400 mg Single Dose (N = 243)** | **FDC 400 mg ALB - 18/9 mg IVM** | |
|---|---|---|---|
| | | **Single Dose (N = 381)** | **Daily Dose x3 Days (N = 377)** |
| **First and second clinical trial Pooled** | | | |
| Number of eggs per gram (EPG) at pre-treatment^{a} | | | |
| n | 131 | 251 | 254 |
| Geometric mean | 124.4 | 126.8 | 128.8 |
| Geometric CV% | 262.4 | 253.3 | 281.5 |

| Number of eggs per gram (EPG) at post-treatment^{a} | | | |
|---|---|---|---|
| n | 131 | 251 | 254 |
| Geometric mean | 21.5 | 2.0 | 1.1 |
| Geometric CV% | 2936.6 | 326.7 | 94.0 |
| **Geometric mean ERR for *T. trichiura* (%)** | 83.4 | 99.2 | 99.9 |

| Difference in logarithm of EPG at post-treatment (vs. ALB 400 mg Single Dose) | | | |
|---|---|---|---|
| LS mean difference | - | -2.4 | -2.9 |
| P-value | - | < 0.0001 | < 0.0001 |

| | | | |
|---|---|---|---|
| Abbreviations: ALB=albendazole; CV=coefficient of variation; FDC=fixed-dose co-formulation (ALB/IVM); EPG=number of eggs per gram; ERR=egg reduction rate; ITT=intent-to-treat; IVM=ivermectin; LS=least squares. a. Geometric means are calculated with 1 added to all egg counts to remove zero values (EPG +1). Geometric mean ERR is calculated as (1 - geometric mean egg counts at post-treatment/geometric mean egg counts at pre-treatment)*100. Notes: Differences and p-values are calculated using an analysis of covariance (ANCOVA), in which the logarithm of the egg count at post-treatment is the dependent variable; site and treatment are fixed effect; and the logarithm of the egg count at pre-treatment is the covariate. Participants missing the post-treatment stool sample were considered cured. Participants withdrawn before Visit 6 (21 days after treatment) with a stool sample were included in the analysis. | | | |

The CR for *T. trichiura* was also analysed by qPCR and the results were consistent with the primary analysis using microscopy, significantly higher CRs for the FDC x3 arm (81.1%) and FDC Single Dose arm (68.4%) compared with the ALB arm (46.0%; p < 0.0001 for both comparisons) (Table 3).

Among participants positive for *T. trichiura* infection by qPCR before treatment, parasite burden after treatment was significantly reduced compared with pre-treatment in the FDC arms, and in the ALB arm (p < 0.0001 for the pre-treatment to post-treatment comparisons of Ct-values across all 3 study arms).

**Table 3. Cure Rate for Participants Infected with T. trichiura at 21 Days After Treatment by qPCR (first and second clinical trial ITT Population)**

| | **ALB 400 mg Single Dose (N = 243)** | | **FDC** | | |
|---|---|---|---|---|---|
| | | | **400 mg ALB - 18/9 mg IVM** | | |
| | | | **Single Dose (N = 381)** | | **Daily Dose x3 Days (N = 377)** |
| ***T. trichiura*** | | | | | |
| Number of participants positive for infection at pre-treatment, n | 126 | | 237 | | 244 |
| Number of participants cured at post-treatment, n | 58 | | 162 | | 198 |
| **Cure rate (CR),** % (95% Cl) | 46.0 (37.3, 54.7) | | 68.4 (62.4, 74.3) | | 81.1 (76.2, 86.1) |

| Difference in CR (vs. ALB 400 mg Single Dose) | | | | | |
|---|---|---|---|---|---|
| Difference^{a} | | 22.9 | | 35.2 | |
| P-value^{b} | | < 0.0001 | | < 0.0001 | |

| | | | | | |
|---|---|---|---|---|---|
| Abbreviations: ALB=albendazole; FDC=fixed-dose co-formulation (ALB/IVM); ITT=intent-to-treat; IVM=ivermectin; qPCR=quantitative polymerase chain reaction; STH=soil-transmitted helminth. a. Difference in cure rates, expressed in percentages, and based on Mantel Haenszel methods to account for stratification by site. | | | | | |

Regarding the secondary efficacy endpoints, the CRs and ERRs for *hookworm* were significantly higher for the FDC x3 arm compared with the ALB arm (p < 0.0001 for all comparisons). FDC Single Dose demonstrated activity against *hookworm,* with CR similar to that observed with ALB (Table 4 and 5).

**Table 4. Cure Rate for Participants Infected with Hookworm at 21 Days After Treatment by Microscopy (first and second clinical trial ITT Population)**

| | | FDC 400 mg ALB - 18/9 mg IVM | |
|---|---|---|---|
| | **ALB 400 mg Single Dose (N = 243** | **Single Dose (N = 381)** | **Daily Dose x3 Days (N = 377)** |
| First and second clinical trial Pooled | | | |
| **Number of participants positive for infection with hookworm at pre-treatment, n** | 108 | 128 | 124 |
| **Number of participants cured at post-treatment, n** | 70 | 99 | 117 |
| Cure rate (CR), **% (95% Cl)** | 64.8 (55.8, 73.8) | 77.3 (70.1, 84.6) | 94.4 (90.3, 98.4) |
| **Difference in CR (vs. ALB 400 mg Single Dose)** | | | |
| **Difference^{a}** | | 12.7 | 29.3 |
| **P-value^{b}** | | 0.0321 | < 0.0001 |

| | | | |
|---|---|---|---|
| Abbreviations: ALB=albendazole; FDC=fixed-dose co-formulation (ALB/IVM); ITT=intent-to-treat; IVM=ivermectin a. Differences in cure rates are expressed in percentages and are based on Mantel Haenszel methods to account for stratification by site. b. The p-values are based on the Cochran-Mantel-Haenszel test, controlling for the effect of site. Notes: A participant with multiple infections was included in the analysis of each target species that the participant was infected with. Participants missing the post-treatment stool sample were considered as not cured. Participants withdrawn before Visit 6 (21 days after treatment) with stool sample were included in the analysis. | | | |

**Table 5. The Egg Reduction Rate of Hookworm at 21 Days After Treatment by Microscopy (first and second clinical trial ITT Population)**

| | | **FDC 400 mg ALB - 18/9 mg IVM** | |
|---|---|---|---|
| | **ALB 400 mg Single Dose (N = 243)** | **Single Dose (N = 381)** | **Daily Dose x3 Days (N = 377)** |
| **First and second clinical trial Pooled** | | | |
| Number of eggs per gram (EPG) at pre-treatment^{a} | | | |
| n | 108 | 128 | 124 |
| Geometric mean | 114.4 | 123.9 | 112.2 |
| Geometric CV% | 201.7 | 206.0 | 267.5 |

| Number of eggs per gram (EPG) at post-treatment^{a} | | | |
|---|---|---|---|
| n | 108 | 128 | 124 |
| Geometric mean | 3.5 | 2.4 | 1.3 |
| Geometric CV% | 548.6 | 417.9 | 150.5 |
| **Geometric mean ERR for hookworm (%)** | 97.8 | 98.8 | 99.7 |
| | | | |

| Difference in logarithm of EPG at post-treatment (vs. ALB 400 mg Single Dose) | | | |
|---|---|---|---|
| LS mean difference | - | -0.4 | -1.0 |
| P-value | - | 0.0460 | < 0.0001 |

| | | | |
|---|---|---|---|
| Abbreviations: ALB=albendazole; CV=coefficient of variation; FDC=fixed-dose co-formulation (ALB/IVM); EPG=number of eggs per gram; ERR=egg reduction rate; ITT=intent-to-treat; IVM=ivermectin; LS=least squares. a. Geometric means are calculated with 1 added to all egg counts to remove zero values (EPG +1). Geometric mean ERR is calculated as (1 - geometric mean egg counts at post-treatment/geometric mean egg counts at pre-treatment)*100. Differences and p-values are calculated using an analysis of covariance (ANCOVA), in which the logarithm of the egg count at post-treatment was the dependent variable, site and treatment are fixed effect, and the logarithm of the egg count at pre-treatment is the covariate. Participants missing the post-treatment stool sample were considered cured. Participants withdrawn before Visit 6 (21 days after treatment) with a stool sample were included in the analysis. | | | |

Efficacy of FDC against *S*. *stercoralis* is shown in the table below.

**Table 6. Cure Rate for Participants Infected with S. stercoralis at 21 Days After Treatment by Microscopy (first and second clinical trial ITT Population).**

| | | **FDC 400 mg ALB - 18/9 mg IVM** | |
|---|---|---|---|
| | **ALB 400 mg Single Dose (N = 243)** | **Single Dose (N = 381)** | **Daily Dose x3 Days (N = 377)** |
| **First and second clinical trial Pooled** | | | |
| Number of participants positive for infection with S. *stercoralis* at pre-treatment, n | 16 | 44 | 44 |
| Number of participants cured at post-treatment, n | 13 | 40 | 43 |
| **Cure rate (CR),** % (95% Cl) | 81.3 (62.1, 100.0) | 90.9 (82.4, 99.4) | 97.7 (93.3, 100.0) |

| | | | |
|---|---|---|---|
| Abbreviations: ALB=albendazole; FDC=fixed-dose co-formulation (ALB/IVM); ITT=intent-to-treat; IVM=ivermectin Notes: A participant with multiple infections was included in the analysis of each target species that the participant was infected with. | | | |

Participants missing the post-treatment stool sample were considered as not cured.

Participants withdrawn before Visit 6 (21 days after treatment) with stool sample were included in the analysis.

Moreover, this second clinical trial contained Exploratory Objectives to evaluate the efficacy of FDC single dose and FDC x3 compared to single dose ALB against *A. lumbricoides* in participants co-infected with the STH and to describe the efficacy of FDC single dose and FDC x3 in the prevalence of scabies compared to single dose ALB 400 mg.

**Table 7. Cure Rate for A. lumbricoides in Co-Infected Participants at 21 Days after Treatment by Microscopy (first and second clinical trial ITT Population)**

| | | **FDC 400 mg ALB - 18/9 mg IVM** | |
|---|---|---|---|
| | **ALB 400 mg Single Dose (N = 243)** | **Single Dose (N = 381)** | **Daily Dose x3 Days (N = 377)** |
| **First and second clinical trial Pooled** | | | |
| Number of participants positive for infection with *A lumbricoides* at pre-treatment, n | 15 | 21 | 25 |
| Number of participants cured at post-treatment, n | 15 | 21 | 25 |
| **Cure rate (CR),** % (95% Cl) | 100.0 (100.0, 100.0) | 100.0 (100.0, 100.0) | 100.0 (100.0, 100.0) |

| Difference in CR (vs. ALB 400 mg Single Dose) | | | |
|---|---|---|---|
| Difference^{a} | - | NE | NE |
| P-value^{b} | - | NE | NE |

| | | | |
|---|---|---|---|
| Abbreviations: ALB=albendazole; FDC=fixed-dose co-formulation (ALB/IVM); ITT=intent-to-treat; IVM=ivermectin; NE=not evaluable a. Differences in cure rates are expressed in percentages and based on Mantel Haenszel methods to account for stratification by site. b. The p-values are based on the Cochran-Mantel-Haenszel test, controlling for the effect of site. Notes: A participant with multiple infections was included in the analysis of each target species that the participant was infected with. Participants missing the post-treatment stool sample were considered as not cured. Participants withdrawn before Visit 6 (21 days after treatment) with stool sample were included in the analysis. | | | |

**Table 8. Egg Reduction Rate for A. lumbricoides in Co-Infected Participants at 21 Days After Treatment by Microscopy (first and second clinical trial ITT Population)**

| | | **FDC 400 mg ALB - 18/9 mg IVM** | |
|---|---|---|---|
| | **ALB 400 mg Single Dose (N = 243)** | **Single Dose (N = 381)** | **Daily Dose x3 Days (N = 377)** |
| **Second clinical trial** | | | |
| Number of eggs per cell (EPG) at pre-treatment^{a} | | | |
| n | 15 | 21 | 25 |
| Geometric mean | 5070.3 | 3523.2 | 3744.0 |
| Geometric CV% | 519.4 | 572.9 | 712.1 |

| Number of eggs per cell (EPG) at post-treatment^{a} | | | |
|---|---|---|---|
| n | 15 | 21 | 25 |
| Geometric mean | 1.0 | 1.0 | 1.0 |
| Geometric CV% | 0.0 | 0.0 | 0.0 |
| **Geometric mean ERR for *A*. *lumbricoides* (%)** | 100.0 | 100.0 | 100.0 |

| Difference in logarithm of EPG at post-treatment (vs. ALB 400 mg Single Dose) | | | |
|---|---|---|---|
| LS mean difference | - | 0.0 | 0.0 |
| P-value | - | NE | NE |

| | | | |
|---|---|---|---|
| Abbreviations: ALB=albendazole; CV=coefficient of variation; FDC=fixed-dose co-formulation (ALB/IVM); EPG=number of eggs per gram; ERR=egg reduction rate; ITT=intent-to-treat; IVM=ivermectin; LS=least squares; NE=not evaluable. a. Geometric means are calculated with 1 added to all egg counts to remove zero values (EPG +1). Geometric mean ERR is calculated as (1 - geometric mean egg counts at post-treatment/geometric mean egg counts at pre-treatment)*100. | | | |

Results of the sensitivity analysis using the first and second clinical trial Efficacy Per-protocol population (all randomized participants who received at least 1 dose of IMP, did not withdraw, were without major protocol deviations, and were not lost to follow-up) were consistent with the primary analysis using the ITT population and support the primary efficacy result.

Subgroup analyses showed no effect for IVM drug exposure (> 400 µg/kg; ≤ 400 µg/kg) or worm burden (light; moderate) on the efficacy demonstrated by FDC, with CR results in these subgroups consistent with the results of the primary analysis (p < 0.0167 for all comparisons between FDC arms and ALB arm).

In summary this first and second clinical trial met its primary objectives of demonstrating safety (First clinical trial) and efficacy (Second clinical trial) of FDC in the enrolled paediatric populations:
- FDC is more effective for curing *T. trichiura* infections than ALB, as shown by the significantly higher CRs compared with ALB, and supported by the secondary efficacy endpoint result of significantly higher *T. trichiura* ERRs compared with ALB.
- Both FDC administration schedules are considered viable dosing strategies, as shown by significantly higher *T. trichiura* CRs with both the FDC Single Dose and FDC x3 compared with ALB.
- FDC x3 is more effective for curing *hookworm* infection than ALB.
- The efficacy of FDC for curing *S*. *stercoralis* is shown in several patients.
- Results of the PPK analysis indicate that the FDC single dose and FDC x3 dosing regimens are adequate for participants over or under 30 kg.
- Both FDC single dose, and FDC administered as a single daily dose for 3 consecutive days, are safe and tolerable in paediatric populations, as shown by no serious TEAEs and only mild or moderate TEAEs.

## Claims

1. An oral pharmaceutical composition in the form of a fixed-dose combination comprising ivermectin, a benzimidazole carbamate and at least one pharmaceutically acceptable excipient, wherein said oral pharmaceutical composition comprises ivermectin or a pharmaceutically acceptable salt or solvate thereof in an amount of 5 to 25 mg.

2. The oral pharmaceutical composition according to claim 1 which is an immediate-release pharmaceutical composition.

3. The oral pharmaceutical composition according to any one of the preceding claims, wherein said composition comprises ivermectin or a pharmaceutically acceptable salt or solvate thereof in an amount of 5 to 25 mg, more preferably 7 to 20 mg, more preferably 8 to 19 mg, even more preferably 9 to 18 mg, or most preferably 9 or 18 mg.

4. The oral pharmaceutical composition according to any one of the preceding claims, wherein said composition comprises ivermectin in an amount of about 0.5% to about 3%, for example about 0.5% to about 2%, or about 0.6% to about 1.8%, or about 0.75% to 1.5% by weight relative to the total weight of the oral pharmaceutical composition.

5. The oral pharmaceutical composition according to any one of the preceding claims, wherein said composition comprises the benzimidazole carbamate or a pharmaceutically acceptable salt or solvate thereof in an amount of 200 to 800 mg, more preferably 250 to 600 mg, even more preferably 350 to 450 mg, or most preferably 400 mg.

6. The oral pharmaceutical composition according to any one of the preceding claims, wherein said composition comprises the benzimidazole carbamate in an amount of about 0.5% to about 70%, for example about 10% to about 60%, or about 15% to about 45%, or about 25% to 35% by weight relative to the total weight of the oral solid pharmaceutical composition.

7. The oral pharmaceutical composition according to any one of the preceding claims, wherein the at least one excipient is selected from:
(i) binders/fillers such as mannitol, crospovidone, polyvinyl acetate, povidone, croscarmellose sodium, povidone-30K, starch, microcrystalline cellulose, lactose, dicalcium phosphate, and or combinations thereof;
(ii) disintegrants such as starch, croscarmellose sodium, sodium starch glycolate, and cross-linked polyvinyl pyrrolidone;
(iii) glidants such as silica and talc;
(iv) lubricants such as stearic acid, magnesium stearate, and sodium stearyl fumarate;
(v) sequestering agents and/or chelating agents such as ethylene diamine tetraacetic acid (EDTA), acetate or anhydrous citric acid;
(vi) colorants such as FD&C lake pigments;
(vii) flavoring agents such as natural and artificial flavors;
(viii) preservatives such as benzoic acid;
(ix) antioxidants such as butylated hydroxyanisole, butylated hydroxytolulene; and citric acid; and
(x) pH modifiers such as citric acid and fumaric acid.

8. The oral pharmaceutical composition according to any one of the preceding claims, wherein the pharmaceutical composition comprises at least one excipient selected from the group consisting of mannitol and/or croscarmellose sodium, wherein the total amount of mannitol and/or croscarmellose sodium is in a range of 37 to 88 wt% of the total weight of the pharmaceutical composition, preferably in a range of 47 to 78 wt% of the total weight of the pharmaceutical composition, more preferably in a range of 52 to 77 wt% of the total weight of the pharmaceutical composition and most preferably in a range of 60 to 75 wt% of the total weight of the pharmaceutical composition.

9. The oral pharmaceutical composition according to any one of the preceding claims, wherein the oral pharmaceutical composition comprises ivermectin, albendazole as the benzimidazole carbamate and at least one pharmaceutically acceptable excipient.

10. The oral pharmaceutical composition according to any one of the preceding claims, wherein the oral pharmaceutical composition is an immediate-release pharmaceutical composition, preferably an oro-dispersible tablet.

11. The oral pharmaceutical composition according to any one of the preceding claims for use in a treatment of parasitic infections, preferably soil-transmitted helminths.

12. The oral pharmaceutical composition for use according to claim 11, wherein the pharmaceutical composition is administered once per day for 1 to 3 consecutive days, preferably once on one day or once for three consecutive days.

13. The oral pharmaceutical composition for use according to any one of claims 11 and 12, wherein the pharmaceutical composition comprises ivermectin in an amount of 9 mg or 18 mg and albendazole in an amount of 400 mg and wherein the parasitic infection is caused by one or more parasites selected from the group consisting of *A. lumbricoides, S. stercoralis, T. trichiura* or hookworms such as *Necator americanus* and *Ancylostoma duodenale.*

14. The oral pharmaceutical composition for use according to any one of claims 11 to 13, wherein the total amount of mannitol and/or croscarmellose sodium is in a range of 37 to 88 wt% of the total weight of the pharmaceutical composition, preferably in a range of 47 to 78 wt% of the total weight of the pharmaceutical composition, more preferably in a range of 52 to 77 wt% of the total weight of the pharmaceutical composition and most preferably in a range of 60 to 75 wt% of the total weight of the pharmaceutical composition.

15. A method of producing an oral pharmaceutical composition according to any of the claims 1 to 10 comprising the following steps:
- granulating a mixture of benzimidazole carbamate with at least a part of the binder, if present, and at least a part of the amount of disintegrant, if present;
- sieving the wet mass obtained in the granulation step;
- separately adding ivermectin and the remaining part of the binder, if present, other excipients, if present, and the remaining part of the amount of disintegrant, if present, into a blender;
- mixing ivermectin and the other components to form a blend_{IVM};
- optionally sieving the blend_{IVM} comprising ivermectin by using a mesh of between 0.250 mm and 0.850 mm;
- blending the sieved composition comprising the benzimidazole carbamate, with the optionally sieved blend_{IVM} comprising ivermectin and the remaining excipients to be used;
- lubricating the mixture obtained in the blend step; and
- granulating or compressing the lubricated mixture to produce a final dosage form.
